# EUROPEAN PATENT APPLICATION

(11) **EP 2 216 049 A1**
(43) Date of publication of application: **11.08.2010**
(21) Application number: 09001693.2
(22) Date of filing: 06.02.2009
(51) Int. Cl.: A61K 47/48

(54) **Drug conjugates with polyglycerols**

(71) Applicant: Freie Universität Berlin, 14195 Berlin (DE); KTB Tumorforschungsgesellschaft mbH, 79106 Freiburg (DE)
(72) Inventor: The designation of the inventor has not yet been filed
(74) Representative: Müller-Boré & Partner Patentanwälte

(57) **Abstract**

The present invention relates to a drug polymer conjugate comprising a pharmaceutically and/or diagnostically active compound bound to a dendritic polyglycerol core having a polyethylene glycol shell.

## Description

The present invention relates to a drug polymer conjugate comprising a pharmaceutically and/or diagnostically active compound bound to a dendritic polyglycerol core having a polyethylene glycol shell.

Most of the drugs used at present are compounds having low molecular weights and exhibit, when systemically administered to a patient, a high plasma clearance or total body clearance. Furthermore, said low molecular weight compounds show a high tendency to penetrate body tissues by diffusion, resulting in a uniform biodistribution. These are the two main reasons why only small quantities of the drug reach the site of action and, due to distribution over healthy tissues of the body, said drugs give rise to problematic side-effects. These disadvantages are of particular concern for those drugs having a high cytotoxic potential, such as cytotoxic agents, immunosuppressive agents or virostatic agents.

Several strategies have been pursued for improving the selectivity of low molecular weight drugs and thus to increase the concentration of the active agent in the desired tissue, while the concentration of the same is decreased in healthy tissues for a reduction of side-effects. These include active and passive targeting approaches with antibodies, serum proteins, synthetic polymers, liposomes and nanocarriers (R. Haag, F. Kratz (2006): Polymer Therapeutics: Concepts and Applications, Angewandte Chemie, Int. Ed., 45, 1198-1215). In the design of drug conjugates with synthetic polymers there is a great need of using polymers with a low polydispersity and high uniformity, high water-solubility and well defined functional groups for drug conjugation.

In view of the above, the technical problem underlying the present invention is to provide novel prodrugs which should comprise a suitable carrier having at least one, and preferably two or more binding site(s) and one or more pharmaceutically and/or diagnostically active compounds bound thereto, whereby improved therapeutic properties should be obtained.

According to the present invention, the above-described technical problem is solved by providing a drug polymer conjugate, comprising a dendritic polyglycerol core with a polyethylene glycol shell and at least one pharmaceutically and/or diagnostically active compound.

The expression "drug polymer conjugate" of the present invention is not specifically restricted and includes any compound wherein a pharmaceutically and/or diagnostically active compound is bound to a dendritic polyglycerol. In this context, the term "dendritic polyglycerol" as used herein includes any substance which contains at least two glycerol units in its molecule and wherein said molecule is characterized by a branched structure. According to the present invention, the term "glycerol unit" does not only relate to glycerol itself but also includes any subunits which are based on glycerol, such as for example

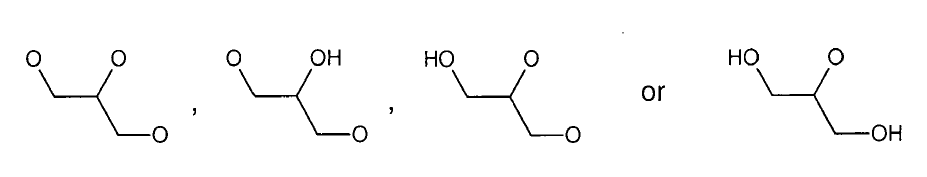

Preferably, the dendritic polyglycerol includes three or more, more preferably four or more and most preferably five or more of said glycerol units, either directly bound to each other, in form of different parts of a molecule or as a combination of both. The dendritic polyglycerol structure can be obtained by either a perfect dendrimer synthesis, a hyperbranched polymer synthesis or a combination of both (R. Haag, A. Sunder, J.-F. Stumbé, (2000): An approach to glycerol dendrimers and pseudo-dendritic polyglycerols, J. Am. Chem. Soc. 122, 2954-2955). The dendritic polyglycerol can also be further functionalized with various reactive goups, such as amines, halides, hydrazines, sulfonates, thiols etc. (S. Roller, H. Zhou, R. Haag (2005): High-loading polyglycerol supported reagents for Mitsunobu and acylation reactions and other useful polyglycerol derivatives Molecular Diversity, 9, 305-316).

The expression "core" as used herein is not specifically restricted and relates to the central or inner part of a molecular structure having for example a spherical or cylindrical.

The expression "polyethylene glycol" means, according to the present invention, any molecule which contains at least two ethylene glycol units, i.e. 1,2-ethanediol units, and is not restricted in any other way. For example, the term polyethylene glycol includes oligoethylene glycols having 2 to 12 ethylene glycol units, or polyethylene glycols having up to several hundred ethylene glycol units. Moreover, according to the present invention, the expression polyethylene glycol also includes mixtures of polyethylene glycol groups comprising different numbers of ethylene glycol units. Additionally, the polyethylene glycols of the present invention are not limited to polyethylene glycol as such but may contain one or more other chemical groups which may, for example, add functionality to the polyethylene glycol such as binding or cleavage sites. Further examples of such chemical groups are functional end groups or groups which originate from the synthesis of said polyethylene glycols or from purification and isolation processes.

According to the present invention, said polyethylene glycol comprises from 2 to 1000, preferably from 3 to 500 and more preferably from 4 to 100 ethylene glycol units.

The term "shell" as used herein relates to a portion of a molecule or a conjugated set of molecules which is in a more peripheral position in respect to a core or central portion of the same molecule. The polyethylene glycol shell of the present application may consist of only one type of polyethylene glycol molecules, but may also consist of a variety of different polyethylene glycols. For example, the polyethylene glycol shell of the present invention may contain a distribution over different polyethylene glycol molecules, as e.g. present in commercially available polyethylene glycols, wherein the mean molecular weight is given.

The term "prodrug" as used herein relates to certain forms of the drug polymer conjugate of the present invention and means any form thereof which can be administered to an organism, such as a human, in an inactive or less active form and is converted, e.g. by metabolization, into the active form. Said conversion of the prodrug into the active form is not specifically restricted and includes any chemical and/or physical alteration of the prodrug which occurs after administration, such as for example release of an active part of the prodrug at the location of action.

The expression "pharmaceutically and/or diagnostically active compound" means any compound which brings about a pharmacological effect either by itself or after its conversion in the organism in question, and thus also includes the derivatives from these conversions. The pharmacological effect of the pharmaceutically and/or diagnostically active compound according to the present invention can be a single effect only, e.g. a cytostatic effect, or a broad pharmacological spectrum of action, such as a cytostatic and antiphlogistic effect at the same time.

According to one embodiment of the present invention, there is provided a drug polymer conjugate as defined above, wherein the pharmaceutically and/or diagnostically active compound is bound to said dendritic polyglycerol core through a cleavable linker.

The expression "bound to said dendritic polyglycerol core" is not specifically limited and does not exclude the binding of the pharmaceutically and/or diagnostically active compound to the polyethylene glycol shell.

The expression "cleavable linker" means any linker which can be cleaved physically or chemically. Examples for physical cleavage may be cleavage by light, radioactive emission or heat, while examples for chemical cleavage include cleavage by redox-reactions, hydrolysis, pH-dependent cleavage or cleavage by enzymes.

Examples of said linkers include cleavable linkers comprising one or more hydrolytically cleavable bonds, the hydrolysis of which releases the pharmaceutically and/or diagnostically active compounds. Examples for hydrolytically cleavable bonds are ester bonds or metal-complex bonds, such as are present in platinum-dicarboxylate complexes, where a diaminediaquoplatinum(II) complex is liberated. Furthermore, the cleavable linker may be cleavable by an enzyme. For example, the cleavable linker of the present invention may contain at least one peptide bond which preferably lies within a cleavable peptide sequence of a protease. At least one peptide bond can therefore be implemented by the insertion of a respective peptide sequence into the cleavable linker. The cleavabe linker may also contain one or more self-immolative linkers that produces after peptide cleavage a labile self-immolative spacer drug derivative that in turn hydrolyses in a spontaneous reaction and releases the pharmaceutically and/or diagnostically active compound. One example of a self-immolative linker is a *p*-aminobenzyloxycarbonyl (PABC) spacer or any of the following wherein R is a functional group selected from H, Cl, Br, I, F, NO₂, CN, OH or an aliphatic or aromatic moiety and the trigger is a peptide that acts as a protease substrate. Specific examples of such a linker are listed in the following Table 1:

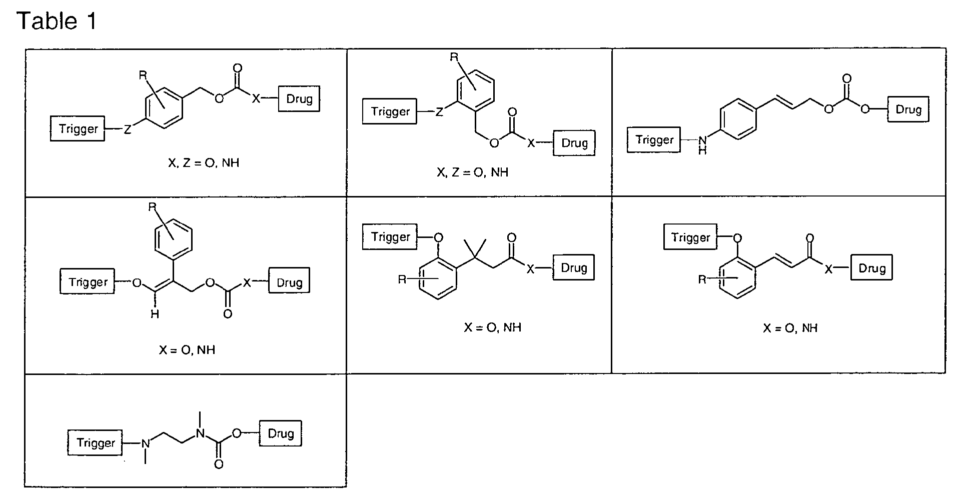

Suitable enzymes are, for example, proteases and peptidases, for example matrix metalloproteases (MMP), cysteine proteases, serine proteases and plasmin activators, which are formed or activated in intensified manner in diseases such as rheumatoid arthritis or cancer, leading to excessive tissue degradation, inflammations and metastasis. Preferred examples of proteases according to the present invention are in particular MMP2, MMP3 and MMP9. Furthermore, the cleavable linker according to the present invention preferably contains at least one acid-labile bond. Examples of acid-labile bonds are ester, acetal, ketal, imine, hydrazone, carboxylhydrazone and sulfonylhydrazone bonds and bonds containing a trityl group.

Another embodiment of the present invention relates to a drug polymer conjugate as defined above, wherein the pharmaceutically and/or diagnostically active compound is bound to said dendritic polyglycerol core through a polymer-binding spacer comprising a polymer-binding moiety and a cleavable linker.

The expression "polymer-binding spacer" as used herein is not specifically restricted as long as it comprises a polymer binding moiety and a cleavable linker. The polymer-binding spacer may therefore be any molecule which is on the one hand capable of binding to a polymer and on the other hand capable of binding to said pharmaceutically and/or diagnostically active compound, and which can be cleaved in order to separate or release the active compound from the polymeric carrier.

According to another embodiment of the present invention, there is provided a drug polymer conjugate as defined above, wherein the at least one pharmaceutically and/or diagnostically active compound is selected from the group consisting of a cytostatic agent, a cytokine, an immunosuppressant, an antirheumatic, an antiphlogistic, an antibiotic, an analgesic, a virostatic, and an antimycotic agent, a transcription factor inhibitor, a cell cycle modulator, an MDR modulator, a proteasome or protease inhibitor, an apoptosis modulator, an enzyme inhibitor, an angiogenesis inhibitor, a hormone or hormone derivative, a radioactive substance, a light emitting substance, and a light absorbing substance.

A specific embodiment of the present invention relates to the above-defined drug polymer conjugate, wherein the at least one pharmaceutically and/or diagnostically active compound is a cytostatic agent selected from the group consisting of N-nitrosoureas; the anthracyclines doxorubicin, daunorubicin, epirubicin, idarubicin, mitoxantrone and ametantrone, 2-pyrollinoanthracyclines, morpholinoanthracyclines, diacetatoxyalkylanthracyclines, and any derivatives thereof; the alkylating agents chlorambucil, bendamustine, melphalan, and oxazaphosphorines, and any derivatives thereof; the antimetabolites 5-fluorouracil, 2'-deoxy-5-fluorouridine, cytarabine, cladribine, fludarabine, pentostatine, gemcitabine and thioguanine, and any derivatives thereof; the folic acid antagonists methotrexate, raltitrexed, pemetrexed and plevitrexed, the taxanes paclitaxel and docetaxel, and any derivatives thereof; the camptothecins topotecan, irinotecan, 9-aminocamptothecin and camptothecin, and any derivatives thereof; the *Vinca* alkaloids vinblastine, vincristine, vindesine and vinorelbine, and any derivatives thereof; calicheamicins and any derivatives thereof; maytansinoids and any derivatives thereof; auristatins and any derivatives thereof; bleomycin, dactinomycin, plicamycin, mitomycin C and *cis*-configured platinum(II) complexes.

In a further embodiment of the above-defined drug polymer conjugate, the pharmaceutically and/or diagnostically active compound comprises one or more radionuclide(s), one or more positron emitter(s), one or more NMR contrast agent(s), one or more fluorescent compound(s), or one or more near infrared contrast agent(s).

According to a another embodiment of the present invention, in the drug polymer conjugate as defined above, said cleavable linker can be cleaved hydrolytically and/or enzymatically and/or pH-dependently.

In one embodiment, the polymer-binding moiety of the above-defined drug polymer conjugate is selected from the group consisting of a maleinimide group, a halogenacetamide group, a halogenacetate group, a pyridylthio group, a vinylcarbonyl group, an aziridin group, a disulfide group, a substituted or unsubstituted acetylene group, and a hydroxysuccinimide ester group.

According to a further embodiment of the present invention, the drug polymer conjugate as defined above is chemically derivatized with at least one polyethylene glycol derivative after drug conjugation.

Another embodiment relates to the above-defined drug polymer conjugate, wherein the polymer-binding moiety is a polyethylene glycol chain and wherein the drug polymer conjugate is in a star-like shape.

According to another aspect of the present invention, there is provided a pharmaceutical composition comprising the above-defined drug polymer conjugate, and optionally a pharmaceutically acceptable carrier and/or a pharmaceutically acceptable adjuvant and/or diluent.

The pharmaceutical composition of the present invention may for example contain solvents and diluents such as physiological saline or a solution containing any pharmaceutically acceptable buffer. Moreover, the pharmaceutical composition of the present invention may be in any form suitable for administration to a patient, for example in an injectable form, as a tablette or a capsule, or as a composition for inhalation.

Moreover, the above-defined pharmaceutical composition can be used in a method for the treatment or prevention of a disorder selected from the group consisting of e.g. cancer, autoimmune diseases, acute or chronic inflammatory diseases or diseases caused by viruses and/or microorganisms.

In a specific embodiment of the present invention, the pharmaceutical composition as defined above is for the treatment or prevention of a disorder selected from the group consisting of cancer, autoimmune diseases, acute or chronic inflammatory diseases and diseases caused by viruses and/or microorganisms.

A further aspect of the present invention relates to a kit comprising the above-defined drug polymer conjugate, or the pharmaceutical composition as defined above, and optionally one or more pharmaceutically acceptable adjuvants and/or diluents.

Another aspect of the present invention relates to the use of the drug polymer conjugate as defined above in the manufacturing of a pharmaceutical composition for treating a patient suffering from a disorder selected from the group consisting of cancer, autoimmune diseases, acute or chronic inflammatory diseases or diseases caused by viruses and/or microorganisms.

Moreover, according to the present invention, one or more of the different above-mentioned pharmaceutically and/or diagnostically active compounds may be bound to the same carrier molecule. Additionally, each of the above-mentioned one or more pharmaceutically and/or diagnostically active compounds may be bound multiple times to the same carrier.

The drug polymer conjugate according to the present invention advantageously comprises one or more pharmaceutically and/or diagnostically active compounds of the same or different kind, thus enabling the combination of a variety of effective compounds at the same time, while side-effects, such as toxicity, are effectively reduced by a polyethylene glycol shell-like structure. Preferably, depending on the number of binding sites within the carrier molecule, the drug polymer conjugate may contain two or more, preferably three or more and more preferably four or more molecules of said pharmaceutically and/or diagnostically active compounds. On the one hand, such a system can advantageously be exploited to yield highly loaded prodrugs comprising in only one of said conjugate molecules a multiplicity of e.g. two, three, four or even more of the same pharmaceutically and/or diagnostically active compound for achieving an enhanced local concentration and thus a surprisingly improved efficacy. On the other hand, prodrugs can be designed which comprise an advantageous combination of different pharmaceutically and/or diagnostically active compounds. By using such drug polymer conjugates comprising combinations of e.g. two, three, four or more different active compounds, highly individualized modes of treatment and/or prevention are provided which allow a simultaneous effect of each of the active compounds to take place.

The above-described highly advantageous effects, namely a high loading, an increased biocompatibility (i.e. low toxicity) due to the polyethylene glycol shell and any desired combination of active compounds can be combined in the drug polymer conjugates of the present invention, thereby providing a surprisingly efficient and versatile tool in treating and/or preventing disorders in a patient such as a human or an animal.

According to the present invention, the unique structure of the drug polymer conjugate enables a surprisingly effective treatment of disorders in humans and animals while a variety of side effects can advantageously reduced to a minimum. The dendritic core of the conjugate according to the present invention allows the binding of a polyethylene glycol shell and of one or more pharmaceutically and/or diagnostically active substances to yield a new class of highly versatile prodrugs. The polyethylene glycole shell ensures a favorable biodistribution and the predetermined breaking point is chemically well-defined because the prodrug is coupled to the dendritic polymer in a final step during syntheis.

The present invention will be further illustrated in the following examples, without any limitation thereto.

The Figures show:

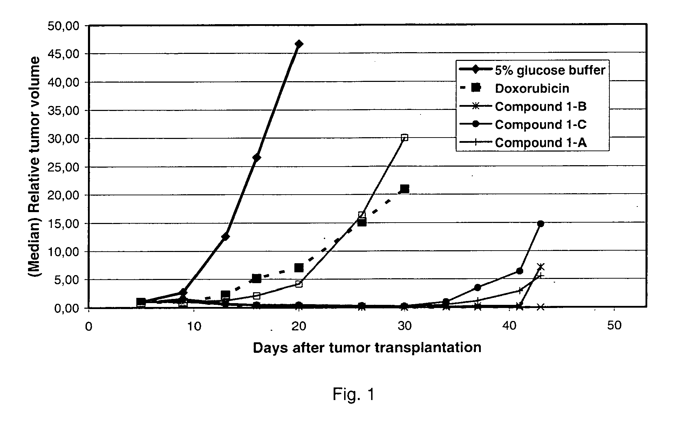

Fig. 1 shows Curves depicting tumor growth inhibition of subcutaneously A2780 xenografts under therapy with doxorubicin and compounds 1-A, 1-B, and 1-C; * significant to control; p < 0.05.

### EXAMPLES

Example 1: Preparation of 4 conjugates with polyglycerolamine (PG 10 kDa and 20% of amine loading) and the (6-maleimidocaproyl)hydrazone derivative of doxorubicin (DOXO-EMCH) using iminothiolane as the thiolating agent and maleimido-polyethylene glycol (PEG 2kDa and 5kDa) as decorating agent - compounds 1-A, 1-B, 1-C and 1-D.

The conjugation reaction was performed at room temperature with vigorous stirring for 90 minutes. To four different flasks, containing 21 mL of a solution of polyglycerolamine (10 mg/mL) in sterile 5 mM EDTA, 50 mM sodium phosphate pH 7.0 were added 43 mL of a solution of 2-iminothiolane (2 mg/mL in the same solvent system). After twenty minutes, a solution of DOXO-EMCH (10 mg/mL in 10 mM sodium phosphate buffer, pH 5.8) was added (a. 8.2 mL; b. 16.4 mL; c. 8.2 mL; d. 1.6 mL). After ten minutes, solutions with a concentration of 200 mg/mL of PEG were added (a. 6.3 mL PEG 2 kDa; b, c and d. 15 mL PEG 5 kDa), and the resulting solutions were stirred for one hour. The solutions were concentrated with CENTRIPREP-10-concentrators from Amicon, FRG (20 min at 4° C and 4000 rpm) to a volume of approximately 5 mL. The PG-DOXO conjugates were purified by gel-filtration through a Sephadex G-25 column (Amersham) with 10 mM sodium phosphate buffer, pH 7.0 yielding 20-50 mL of a red solution. A second concentration with CENTRIPREP was made and finally the conjugates were lyophilized at -60°C for 16 hours to yield a red powder. Yields obtained were between 54% and 74%.

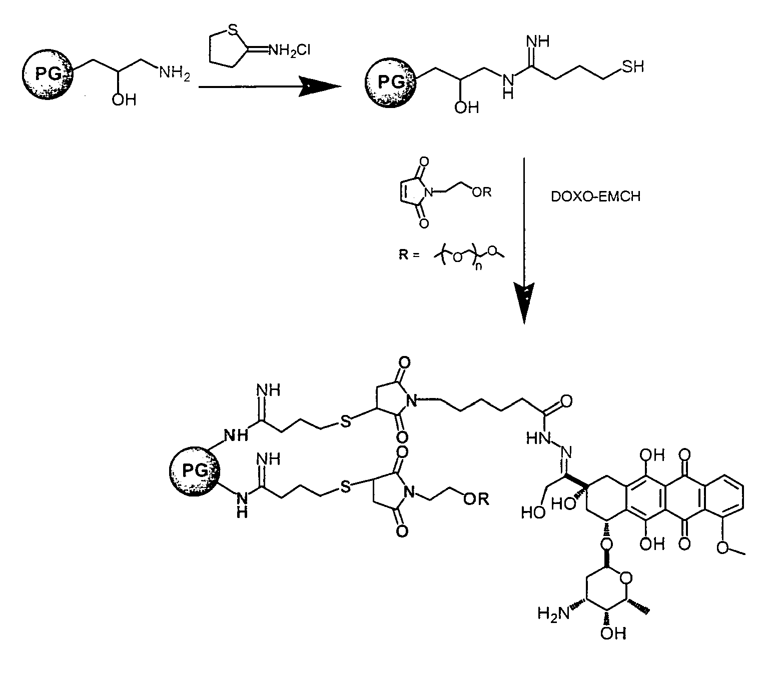

Table 2 summarizes data for the compounds 1-A, 1-B, 1-C and 1-D that were obtained.

**Table 2: Molecular weight, doxorubicin content, yields and structure of compounds 1-A, 1-B, 1-C and 1-D**

| **Sample** | **Theoretical MW** | **Doxorubicin contain in weight %** | **amount recovered/ yield** | **Conjugate - idealized structure** |
|---|---|---|---|---|
| Compound 1-A | ∼ 52 kDa | 3.88 | 718 mg / 54% | 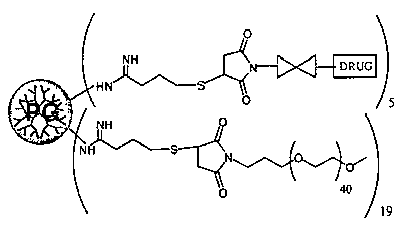 |
| Compound 1-B | ∼ 91 kDa | 2.54 | 2.035 g / 74% | 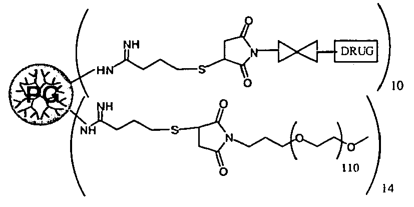 |
| Compound 1-C | ∼ 112 kDa | 1.67 | 1.622 g / 60.75 % | 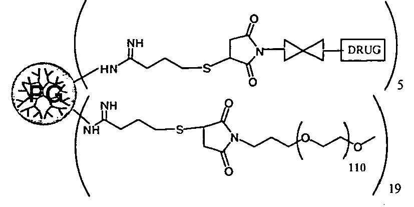 |
| Compound 1-D | ∼ 130 kDa | 0.45 | 1.6678 g / 64% | 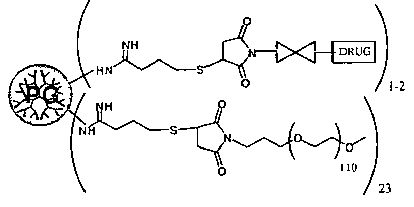 |

Example 2: Preparation of a conjugate with polyglycerolamine (PG 10 kDa and 20% of amine loading) and the (6-maleimidocaproyl)hydrazone derivative of doxorubicin (DOXO-EMCH) using iminothiolane as the thiolating agent and 2-hydroxy ethyl maleimide (HEM) as decorating agent.

The conjugation reaction was performed at room temperature with vigorous stirring for 90 minutes. To 210 mL of a solution of polyglycerolamine (10 mg/mL) in sterile 5 mM EDTA, 50 mM sodium phosphate buffer, pH 7.0 was added 43 mL of a solution of 2-iminothiolane (2 mg/mL in the same solvent system). After twenty minutes, 8.2 mL of a solution of DOXO-EMCH were added (10 mg/mL in buffer 10 mM sodium phosphate, pH 5.8). After ten minutes, 11.1 mL of a solution with a concentration of 10 mg/mL of HEM was added, and the resulting solution was stirred for one hour. The solution was concentrated with CENTRIPREP-10-concentrators from Amicon, FRG (20 min at 4° C and 4000 rpm) to a volume of approximately 5 mL. The PG-DOXO conjugate was purified by gel-filtration through a Sephadex G-25 column (Amersham) with 10 mM sodium phosphate pH 7.0 yielding 20 mL of a red solution. A second concentration with CENTRIPREP was made and finally the conjugate was lyophilized to yield 3.8 mL of solution with a 8.84 mM doxorubicin concentration. Yield was 61 % and the doxorubicin weight percent was 8.11%.

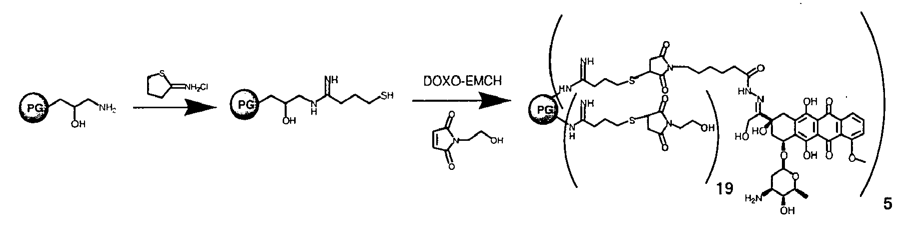

Example 3: Preparation of a conjugate with polyglycerolamine (PG 10 kDa and 7% of amine loading) and the (6-maleimidocaproyl)hydrazone derivative of doxorubicin (DOXO-EMCH) using iminothiolane as the thiolating agent and polyethylene glycol as spacer.

The conjugate was prepared in four steps:

### 1. PEGylation with t-Boc-protected-PEG-N-hydroxysuccinimide ester (NHS-PEG-t-Boc)

To a solution of polyglycerolamine (0.1 g, 0.1013 mmol, 1 eq) in 5 mL DMF the NHS-PEG-*t*-Boc (0,4483 g, 0.1519 mmol, 1.5 eq) was added under vigorous stirring. After 16 hours, DMF was removed through destillation and the crude product was diluted and dialyzed in methanol for 20 hours. The solvent was removed under vacuum to give a white crystalline product. Yield: 64%; a ninhydrin test was made to confirm complete amine reaction.

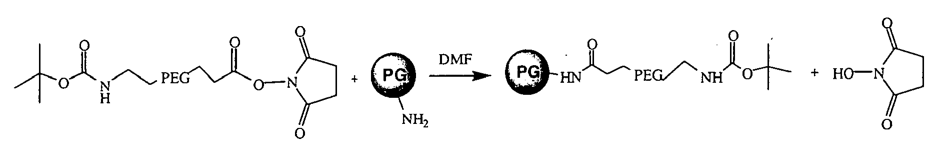

### 2. Amine deprotection of PG-PEG-t-Boc derivate

The PG derivate (t-Boc) obtained from the PEGylation reaction was dissolved in 0.400 mL of a mixture 50:50 of TFA and CH₂Cl₂, and was stirred for 2 h at room temperature. To this solution 5.8 mL sodium phosphate buffer (pH 8, 100 mmolar) and 1.6 mL 1 M NaOH were added to neutralize the solution. The CH₂Cl₂ was removed in vacuo and the resulting aqueous solution was concentrated in CENTRIPREP-10-concentrators from Amicon, FRG (20 min at 4° C and 4000 rpm) and dialyzed in H₂O for 48 h. A ninhydrin test was made to confirm amine formation.

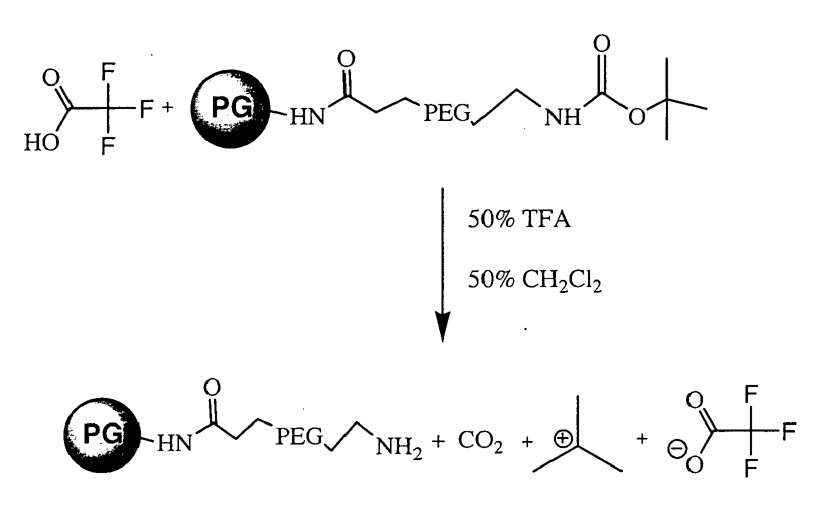

### 3. Activation with 2-iminothiolane (thiol formation)

To a solution of the resulting product of the amine deprotected PG derivate (40 mg, 10 mmol, 1 eq) in sterile buffer (5 mM EDTA, 50 mM sodium phosphate, pH 7.0) were added 0.861 mL of a solution of 2-iminothiolane (2 mg/mL in the same solvent system). After 2 h of stirring the conjugation reaction was performed.

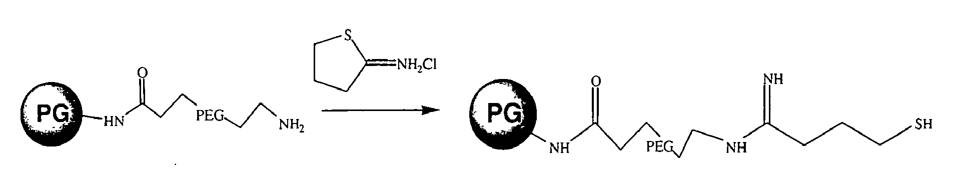

### 4. Conjugation with the (6-maleimidocaproyl) hydrazone derivative of doxorubicin (DOXO-EMCH)

The conjugation reaction was performed at room temperature with vigorous stirring for two hours. To the obtained solution from step 3, 0.787 mL of a solution of DOXO-EMCH was added slowly (10 mg/mL in 10 mM sodium phosphate buffer, pH 5.8). The total amount of prodrug was added in three repeated aliquots, waiting fifteen minutes between each addition. After addition of 10 mM sodium phosphate buffer, pH 7.0 to give a final volume of 15 mL, the solution was concentrated with CENTRIPREP-10-concentrators from Amicon, FRG (20 min at 4° C and 4000 rpm) to a volume of approximately 3 mL. The PG-DOXO conjugate was purified by gel-filtration through a Sephadex G-25 column with 10 mM sodium phosphate buffer, pH 7.0. A second concentration with CENTRIPREP-10-concentrators from Amicon, FRG (20 min at 4° C and 4000 rpm) was made and finally the conjugate was lyophilized to give - 30 mg of a red powder. Yield: 60 %.

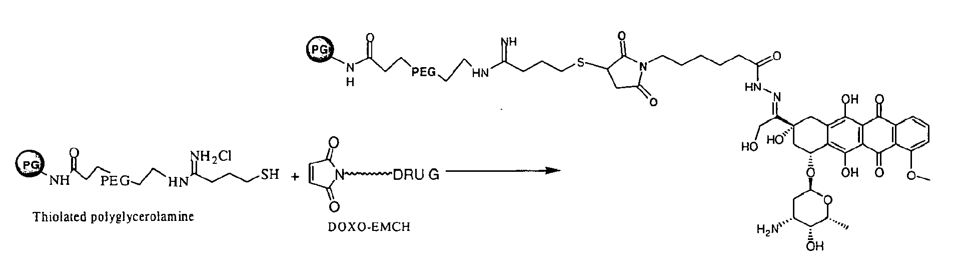

Example 4: Preparation of a conjugate with polyglycerolamine (PG 10 kDa and 7% of amine loading) and the (6-maleimidocaproyl)hydrazone derivative of doxorubicin (DOXO-EMCH) using a polyethylene glycol (PEG) derivate as thiolating agent and spacer.

The conjugate was prepared in three steps:

### 1. PEGylation with (tritylthio)-PEG-N-hydroxysuccinimide ester (NHS-PEG-Trt)

To a solution of polyglycerolamine (0.1 g, 0.1013 mmol, 1 eq) in 5 mL DMF the NHS-PEG-Trt (0,4483 g, 0.1519 mmol, 1.5 eq) was added under vigorous stirring. After 16 hours, DMF was removed through destillation and the crude product was diluted and dialyzed in methanol for 20 hours. The solvent was removed under vacuum to give a white crystalline product. Yield: 60%; a ninhydrin test was made to confirm complete amine reaction.

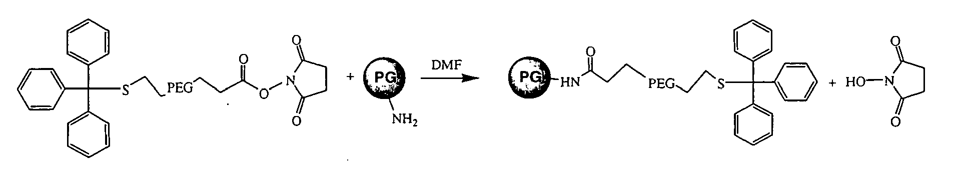

### 2, 3. Thiol deprotection (Trt) and conjugation with the (6-maleimidocaproyl)hydrazone derivative of doxorubicin (DOXO-EMCH)

The PG derivate (Trt) obtained from the PEGylation reaction was dissolved in 0.600 mL of a mixture 95:5 of TFA and tri-isopropyl silane, and was stirred for 2 h at room temperature. 5.8 mL PBS and 1.6 mL NaOH were then added and the solution was concentrated three times with CENTRIPREP-10-concentrators from Amicon, FRG (40, 20, 10 min at room temperature and 4000 rpm). To the resulting solution, 14.5 mL PBS (pH 7, 50 mM, 5 mM EDTA) was added and a second concentration with Centriprep was carried out.

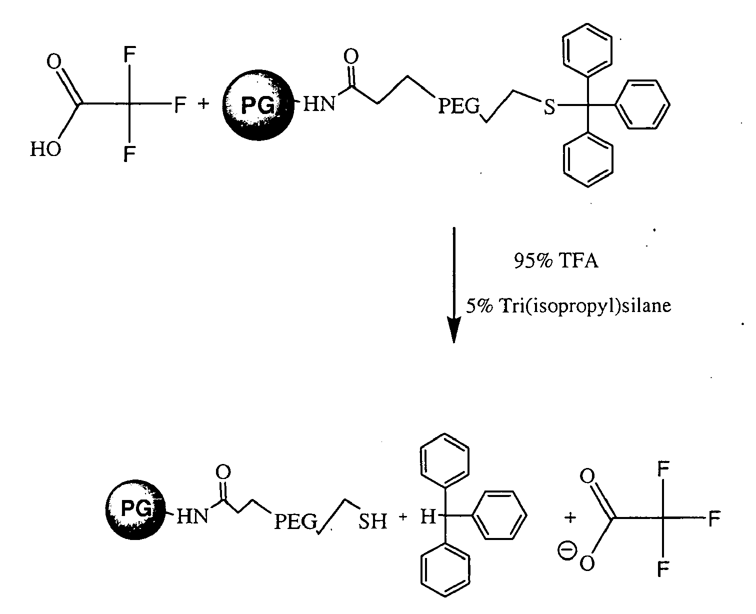

The obtained solution was mixed slowly with 1.967 mL of a DOXO-EMCH solution (2 mg/mL in 10 mM sodium phosphate buffer, pH 5.8). The total amount of prodrug was added in three repeated aliquots, waiting fifteen minutes between each addition. The resulting mixture was stirred for 2 h at room temperature. After addition of 10 mM sodium phosphate, pH 7.0 to give a final volume of 15 mL, the solution was concentrated with CENTRIPREP-10-concentrators from Amicon, FRG (20 min at 4° C and 4000 rpm) to a volume of approximately 3 mL. The PG-DOXO conjugate was purified by gel-filtration through a Sephadex G-25 column with 10 mM sodium phosphate, pH 7.0. A second concentration with Centriprep was made and finally the conjugate was lyophilized to give -22 mg of a red powder. Yield: 50%.

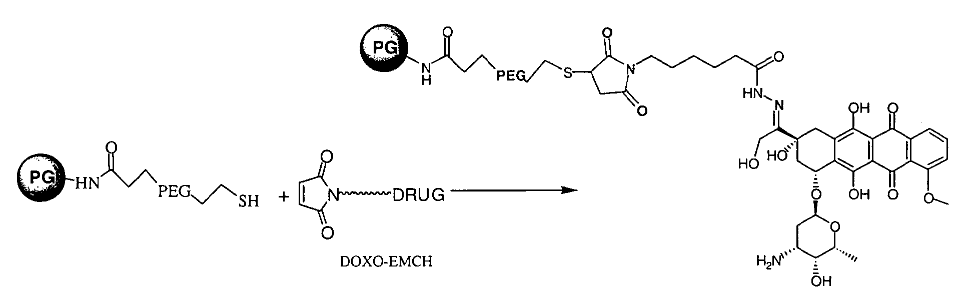

Example 5: pH-dependant stability studies with compounds 1-A, 1-B, 1-C and 1-D at were carried out with HPLC at pH 4 (50 mM sodium acetate buffer) and pH 7.4 (50 mM sodium phosphate buffer). A 730 µM solution of 1-A, 1-B, 1-C or 1-D (concentration stated in doxorubicin equivalents) was incubated at room temperature in the respective buffer system and analyzed by size-exclusion HPLC over 22 h using a Kontron-HPLC system with GeminyxSystem software, column: BioSil SEC250 [300*7,8mm], with a pre-column [80*7,8mm] from Biorad, Germany; flow: 1.2 mL/min, isocratic; injection: 50 µL; mobile phase: 10% acetonitrile / 90% 10 mM sodium phosphate buffer, 0.15 NaCl, ph 7.0, detection at 220 nm and 495 nm.

At pH 4.0 all conjugates 1-A, 1-B, 1-C and 1-D were cleaved and released doxorubicin with the following half-lives:

**Table 4: Half-lives of compounds 1-A, 1-B, 1-C and 1-D at pH 4.0**

| **conjugate** | **t ½ [h]** |
|---|---|
| Compound 1-A | 3.3 |
| Compound 1-B | 2.1 |
| Compound 1-C | 1.9 |
| Compound 1-D | 2.7 |

In contrast, at pH 7.4 all conjugates were stable with less than 10 % release of doxorubicin over 22 h.

Example 6: *In vivo* efficacy studies were carried out with compounds 1-A, 1-B, and 1-C and doxorubicin in the ovarian carcinoma A2780 xenograft model. For the *in vivo* testing, female NMRI: nu/nu mice (Taconic, Denmark) were used. The mice were held in individually ventilaged cages (IVC) under sterile and standardized environmental conditions (25 ± 2 °C room temperature, 50 ± 10 % relative humidity, 12 hour light-dark-rhythm). They received autoclaved food and bedding (ssniff, Soest, Germany) and acidified (pH 4.0) drinking water *ad libitum.* A2780 tumor fragments were transplanted subcutaneously (s.c.) into the left flank region of anaesthetized (40 mg/kg i.p. Radenarkon, Asta Medica, Frankfurt, Germany) mice on day zero. Mice were randomly distributed to the experimental groups (8 mice per group). When the tumors were grown to a palpable size, treatment was initiated. Mice were treated intravenously with either glucose phosphate buffer (10 mM sodium phosphate, 5 % D-(+)-glucose, pH 5.8), doxorubicin (2 x 8 mg/kg), or compounds 1-A, 1-B, and 1-C (3 x 24 mg/kg doxorubicin equivalents, compounds 1-A, 1-B, and 1-C were dissolved in 10 mM sodium phosphate, 5 % D-(+)-glucose, pH 5.8) at weekly intervals. The injection volume was 0.2 mU20 g body weight.

Tumor size was measured twice weekly with a caliper-like instrument in two dimensions. Individual tumor volumes *(V)* were calculated by the formula V = (length x [width]²)/2 and related to the values on the first day of treatment (relative tumor volume, RTV). Statistical analysis was performed with the U-test (Mann and Whitney) with p < 0.05. The body weight of mice was determined every 3 to 4 days.

The results of the experiments in the xenograft tumor models are shown in Fig. 1 and Table 5. The standard and maximum tolerated dose of doxorubicin (2 x 8 mg/kg) was compared to compounds 1-A, 1-B, and 1-C at 3 x 24 mg/kg (doxorubicin equivalents). We and others have shown that 2 x 8 mg/kg doxorubicin is the MTD in nude mice models and higher doses, e.g. 2 x 12 mg/kg, leads to unacceptable toxicity and mortality Kratz et al.: In vitro and in vivo efficacy of acid-sensitive transferrin and albumin doxorubicin conjugates in a human xenograft panel and in the MDA-MB-435 mamma carcinoma model. J. Drug Targeting, 8: 305-318, 2000; Trail et al.: Antigen-specific activity of carcinoma-reactive BR64-doxorubicin conjugates evaluated in vitro and in human tumor xenograft models. Cancer Res., 52: 5693-5700, 1992). In contrast, the compounds 1-A, 1-B, and 1-C could be administered at 3 x 24 mg/kg doxorubicin equivalents without producing mortality or severe body weight loss (see Table 5).

**Table 5: Dose schedule, mortality, body weight change, and antitumor activity of doxorubicin and compounds 1-A, 1-B, and 1-C against human ovarian cancer xenografts (A2780)**

| **Mice** | **Substance** | **Schedule** | **Dose** | **toxic death** | **BWC** | **optimum** **T/C RTV** |
|---|---|---|---|---|---|---|
| | | [days] | [mg/kg/i.v.] | | [%] | [%] |
| 8 | Glucose-phosphate buffer | 6, 13, 20 | | 0 | -2 | |
| 8 | Doxorubicin | 6, 13 | 8 | 0 | -21 (d26) | 15* |
| 8 | Compound 1-A | 6, 13, 20 | 24 | 0 | -15 (d16) | 0* |
| 8 | Compound1-B | 6, 13, 20 | 24 | 0 | -12 (d13) | 1* |
| 8 | Compound 1-C | 6, 13, 20 | 24 | 0 | -6 (d13) | 1* |

| | | | | | | |
|---|---|---|---|---|---|---|
| * significant versus control group - p < 0.05; BWC = body weight change; T/C RTV = relative tumor volume expressed in % treated versus control groups | | | | | | |

With respect to antitumor efficacy, doxorubicin only showed moderate antitumor efficacy. In contrast, treatment with compounds 1-A, 1-B, and 1-C produced excellent antitumor effects with complete tumor remissions up to day 30 (see Fig. 1) and less body weight change (BWC) than for the doxorubicin treated animals (see Table 5).

## Claims

1. A drug polymer conjugate, comprising a dendritic polyglycerol core with a polyethylene glycol shell and at least one pharmaceutically and/or diagnostically active compound.

2. The drug polymer conjugate according to claim 1, wherein the pharmaceutically and/or diagnostically active compound is bound to said dendritic polyglycerol core through a cleavable linker.

3. The drug polymer conjugate according to claim 1, wherein the pharmaceutically and/or diagnostically active compound is bound to said dendritic polyglycerol core through a polymer-binding spacer comprising a polymer-binding moiety and a cleavable linker.

4. The drug polymer conjugate according to any one of claims 1 to 3, wherein the at least one pharmaceutically and/or diagnostically active compound is selected from the group consisting of a cytostatic agent, a cytokine, an immunosuppressant, an antirheumatic, an antiphlogistic, an antibiotic, an analgesic, a virostatic, and an antimycotic agent, a transcription factor inhibitor, a cell cycle modulator, an MDR modulator, a proteasome or protease inhibitor, an apoptosis modulator, an enzyme inhibitor, an angiogenesis inhibitor, a hormone or hormone derivative, a radioactive substance, a light emitting substance, and a light absorbing substance.

5. The drug polymer conjugate according to any one of claims 1 to 4, wherein the at least one pharmaceutically and/or diagnostically active compound is a cytostatic agent selected from the group consisting of N-nitrosoureas; the anthracyclines doxorubicin, daunorubicin, epirubicin, idarubicin, mitoxantrone and ametantrone, 2-pyrollinoanthracyclines, morpholinoanthracyclines, diacetatoxyalkylanthracyclines, and any derivatives thereof; the alkylating agents chlorambucil, bendamustine, melphalan, and oxazaphosphorines, and any derivatives thereof; the antimetabolites 5-fluorouracil, 2'-deoxy-5-fluorouridine, cytarabine, cladribine, fludarabine, pentostatine, gemcitabine and thioguanine, and any derivatives thereof; the folic acid antagonists methotrexate, raltitrexed, pemetrexed and plevitrexed, the taxanes paclitaxel and docetaxel, and any derivatives thereof; the camptothecins topotecan, irinotecan, 9-aminocamptothecin and camptothecin, and any derivatives thereof; the *Vinca* alkaloids vinblastine, vincristine, vindesine and vinorelbine, and any derivatives thereof; calicheamicins and any derivatives thereof; maytansinoids and any derivatives thereof; auristatins and any derivatives thereof; bleomycin, dactinomycin, plicamycin, mitomycin C and *cis*-configured platinum(II) complexes.

6. The drug polymer conjugate according to any one of claims 1 to 5, wherein the pharmaceutically and/or diagnostically active compound comprises one or more radionuclide(s), one or more positron emitter(s), one or more NMR contrast agent(s), one or more fluorescent compound(s), or one or more near infrared contrast agent(s).

7. The drug polymer conjugate according to any one of claims 2 to 6, wherein said cleavable linker can be cleaved hydrolytically and/or enzymatically and/or pH-dependently.

8. The drug polymer conjugate according to any one of claims 3 to 7, wherein the polymer-binding moiety is selected from the group consisting of a maleinimide group, a halogenacetamide group, a halogenacetate group, a pyridylthio group, a vinylcarbonyl group, an aziridin group, a disulfide group, a substituted or unsubstituted acetylene group, and a hydroxysuccinimide ester group.

9. The drug polymer conjugate according to any one of claims 1 to 8, wherein the drug polymer conjugate is chemically derivatized with at least one polyethylene glycol derivative after drug conjugation.

10. The drug polymer conjugate of any one of claims 3 to 9, wherein the polymer-binding moiety is a polyethylene glycol chain and wherein the drug polymer conjugate is in a star-like shape.

11. A pharmaceutical composition comprising the drug polymer conjugate according to any one of claims 1 to 10, and optionally a pharmaceutically acceptable carrier and/or a pharmaceutically acceptable adjuvent and/or diluent.

12. The pharmaceutical composition according to claim 11 for the treatment or prevention of a disorder selected from the group consisting of cancer, autoimmune diseases, acute or chronic inflammatory diseases and diseases caused by viruses and/or microorganisms.

13. A kit comprising the drug polymer conjugate according to any one of claims 1 to 10, or the pharmaceutical composition according to claim 11 or 12, and optionally one or more pharmaceutically acceptable adjuvants and/or diluents.

14. A use of the drug polymer conjugate according to any one of claims 1 to 10 in the manufacturing of a pharmaceutical composition for treating a patient suffering from a disorder selected from the group consisting of cancer, autoimmune diseases, acute or chronic inflammatory diseases or diseases caused by viruses and/or microorganisms.
